# FASCICULE DE BREVET EUROPEEN

(11) **EP 0 546 966 B1**
(45) Date de publication et mention de la délivrance du brevet: **19.07.1995**
(21) Numéro de dépôt: 92420456.3
(22) Date de dépôt: 11.12.1992
(51) Int. Cl.: A61M 39/02

(54) **Raccord pour perfusion et prise d'échantillon**
Verbindungsstück für Infusion und Probenentnahme
Sampling and infusion connection

(30) Priorité: 13.12.1991 IT TO910975
(43) Date de publication de la demande: 16.06.1993
(73) Titulaire: HOSPAL AG, 4008 Basel (CH)
(72) Inventeur: Ragazzi, Guido, I-41035 Massa Finalese (MO) (IT)

(56) Documents cités:
- WO-A-89/00867
- FR-A- 2 322 614
- GB-A- 990 583

## Description

L'invention se rapporte à un raccord pour une ligne de circulation sanguine extracorporelle pour infusion et prise d'échantillon.

Comme on le sait, les lignes pour la circulation sanguine hors du corps, par exemple pour un appareil de dialyse, nécessitent au moins un raccord pour recueillir le sang et un dispositif pour la prise d'échantillon sanguin et/ou pour l'infusion de liquides dans le flux sanguin. Le flux est contrôlé par une pompe, de préférence péristaltique, qui agit au moyen de galets ou équivalent, sur une portion de tuyau flexible du système.

Un raccord connu pour une ligne telle que précitée est décrit dans FR-A-2 322 614 et comprend un corps tubulaire pourvu d'un ouverture pour infusion en aval du flux sanguin et d'une ouverture en amont de prise d'échantillon pour prendre un échantillon sanguin, par exemple par l'intermédiaire d'une seringue. Ce raccord présente l'inconvénient que, lors de la prise d'échantillon, le liquide d'infusion tend à être aspiré à contrecourant par la pression négative causée par la seringue de prise d'échantillon, et à se mélanger au sang prélevé et par conséquent à altérer sa composition.

Par conséquent, afin d'empêcher le liquide infusé d'atteindre l'ouverture de prise d'échantillon au moment où l'aspiration est exercée par la seringue, il serait nécessaire d'interrompre le processus d'infusion pendant un certain temps, avec des conséquences néfastes sur le traitement. Alternativement, le raccord doit être dimensionné de façon à ce que l'ouverture de prise d'échantillon soit disposée à une distance appréciable de l'ouverture pour infusion, auquel cas le raccord sera encombrant et donc l'utilisation peu pratique.

L'invention a pour but la fabrication d'un raccord pour une ligne de circulation sanguine extracorporelle, le raccord étant très simple et dépourvu des inconvénients précités des raccords connus.

Ce but est atteint avec un raccord pour une ligne de circulation sanguine extracorporelle pour infusion et prise d'échantillon, comprenant un corps ayant une cavité tubulaire, dont les deux extrémités sont prévues pour être connectées à deux tuyaux de la ligne, le corps comprenant aussi une ouverture d'infusion pour l'introduction d'un liquide dans la cavité tubulaire, et une ouverture de prise d'échantillon sanguin à partir de la cavité tubulaire, l'ouverture d'infusion étant disposée en aval de l'ouverture de prise d'échantillon par rapport au flux sanguin, caractérisée en ce que la cavité a un étranglement disposé entre les ouvertures, et dont le diamètre diminue progressivement vers l'ouverture pour infusion.

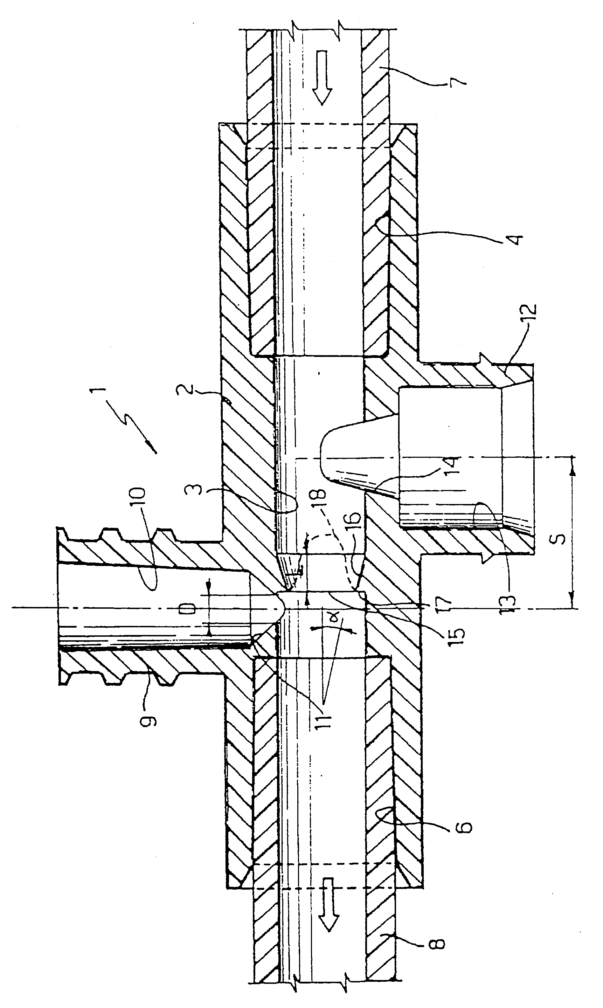

L'invention sera mieux comprise à partir de la description suivante d'un mode de réalisation préféré, donné à titre d'exemple, à l'aide du dessin unique annexé, qui est une coupe transversale d'un raccord pour une ligne de circulation extracorporelle pour infusion et prise d'échantillon.

En référence aux dessins, la référence générale 1 désigne un raccord pour une ligne de circulation sanguine extracorporelle comprenant un corps rigide 2 en matière plastique ayant une cavité tubulaire 3. La cavité 3 a deux extrémités 4, 6 de plus grand diamètre, qui, lors du fonctionnement, sont connectées respectivement à un tuyau d'entrée 7 et à un tuyau de sortie 8 pour le sang, les deux tuyaux faisant partie d'une ligne de circulation extracorporelle (non représentée).

Pendant le fonctionnement, une pompe péristaltique agit sur une portion (non représentée) du tuyau 7 et provoque la circulation du sang en comprimant la portion précitée et en produisant ainsi un flux de pulsations essentiellement sinusoïdal.

Le corps 2 est solidaire d'un manchon 9 ayant une cavité 10 légèrement conique, dont l'axe est perpendiculaire à l'axe de la cavité tubulaire 3. La cavité 10 communique avec la cavité 3 du corps 2 par l'intermédiaire d'une ouverture pour infusion 11 ayant une surface fortement conique, telle que son diamètre D, à l'intersection avec la cavité 3, est d'environ un tiers du diamètre de la cavité 10. Le manchon 9 a un filetage externe destiné au raccord avec un ensemble de tubes pour infusion (non représenté).

Le corps 2 est également solidaire d'un manchon 12 disposé en amont du manchon 9 relativement au sens d'écoulement du flux sanguin. Le manchon 12 a une cavité 13 essentiellement cylindrique, dont l'axe est pareillement perpendiculaire à l'axe de la cavité tubulaire 3, le manchon 12 s'étendant dans la direction opposée au manchon 9. La cavité 13 communique avec la cavité 3 dans le corps 2 par l'intermédiaire d'une ouverture de prise d'échantillon 14 ayant une surface légèrement conique. Le manchon 12 a des moyens conventionnels de fixation pour connecter un bouchon (non représenté) en matériau élastique déformable, prévu pour être percé par une seringue pendant le fonctionnement.

Selon l'invention, la cavité 3 dans le corps 2 est pourvue d'un étranglement (référence générale 15) disposé entre les ouvertures 11 et 14 et ayant un diamètre diminuant progressivement vers l'ouverture pour infusion 11. L'étranglement 15 constitue ainsi une portion d'un tube de Venturi, par exemple la moitié de celui-ci. Plus particulièrement, l'étranglement 15 a une surface tronconique 16, dont la petite base est disposée directement en amont de l'ouverture pour infusion 11. La surface 16 et la cavité 3 forment une marche 17 pouvant avantageusement être une surface annulaire plate orthogonale sensiblement alignée avec le bord de l'ouverture 11 de diamètre D.

La surface 16 est inclinée par rapport à l'axe de la cavité 3 d'un angle α compris entre 10° et 20°, de préférence 15°. La longueur L de l'étranglement 16 est choisie de façon à créer une marche 17 ayant une hauteur comprise entre 1/10 et 3/10 du diamètre de la cavité 3. La distance S entre les axes des deux cavités 10, 13 des manchons 9, 12 est comprise entre une et trois fois le diamètre de la cavité 3 dans le corps 2. Avantageusement, lorsque le diamètre de la cavité 3 est de 4,2 mm, la distance S peut être choisie égale à 8 mm, tandis que la hauteur de la marche 17 peut être choisie égale à 0,5 mm.

Le raccord 1 fonctionne comme suit :
Lorsque la ligne de circulation extracorporelle est utilisée, la pompe péristaltique entraîne le sang au travers de la cavité 3 du corps 2 dans le sens indiqué par la flèche. Le liquide d'infusion est introduit dans la ligne par le manchon 9 et l'ouverture pour infusion 11 du raccord 1. Un effet de tube de Venturi, c'est-à-dire une pression négative, se produit en face de l'étranglement 15 et contribue à l'aspiration du liquide d'infusion par l'ouverture 11 et à son mélange avec le sang provenant du tuyau 7.

Lorsque l'on veut réaliser une prise d'échantillon du sang en circulation, le sang est aspiré en introduisant l'aiguille d'une seringue par le manchon 12 et l'ouverture 14, produisant ainsi une pression négative dans la région en amont de l'étranglement 15. Cependant, la marche 17 et la pression négative résultant de l'effet de tube de Venturi empêchent le liquide d'infusion de se propager dans le sang en amont de la marche 17, et par conséquent le sang prélevé par la seringue n'est pas mélangé au liquide d'infusion.

Au moment où la pompe péristaltique s'arrête temporairement, ralentissant ainsi le flux dans la ligne, on peut faire diffuser le liquide d'infusion vers l'ouverture de prise d'échantillon 14. Cependant, la distance S et la hauteur de la marche 17, lorsqu'elles ont été dimensionnées comme indiqué précédemment, empêchent de façon sûre une telle diffusion en la limitant à une région 18 délimitée par une ligne courbe en traits pointillés sur le dessin.

Ce qui précède montre clairement les avantages du raccord selon l'invention, par rapport aux raccords connus. Le raccord empêche le sang prélevé de se mélanger avec le liquide d'infusion. De plus, la distance S est réduite à un minimum, si bien que le raccord 1 dans son ensemble est très compact.

Bien sûr, plusieurs modifications et perfectionnements peuvent être apportés au raccord 1 décrit, sans s'écarter du domaine des revendications. Par exemple, l'étranglement peut avoir une génératrice courbe au lieu d'une génératrice droite, ce qui évite ainsi l'angle vif entre la surface 16 et la surface de la cavité 3.

## Revendications

1. Raccord pour une ligne de circulation sanguine extracorporelle pour infusion et prise d'échantillon, comprenant un corps (2) ayant une cavité tubulaire (3), dont les deux extrémités (4, 6) sont prévues pour être connectées à deux tuyaux (7, 8) de la ligne, le corps (2) comprenant aussi une ouverture d'infusion (11) pour l'introduction d'un liquide dans la cavité tubulaire (3), et une ouverture (14) de prise d'échantillon sanguin à partir de la cavité tubulaire (3), l'ouverture d'infusion (11) étant disposée en aval de l'ouverture de prise d'échantillon (14) par rapport au flux sanguin, caractérisée en ce que la cavité (3) a un étranglement (15) disposé entre les ouvertures (11, 14), et dont le diamètre diminue progressivement vers l'ouverture pour infusion (11).

2. Raccord selon la revendication 1, caractérisé en ce que l'étranglement (15) comprend au moins une partie d'un tube de Venturi.

3. Raccord selon la revendication 1, caractérisé en ce que l'étranglement (15) a une surface tronconique (16), dont la petite base est sensiblement alignée avec le bord de l'ouverture pour infusion (11).

4. Raccord selon la revendication 3, caractérisé en ce que la surface tronconique (16) est inclinée d'un angle α compris entre 10° et 20° par rapport à l'axe de la cavité (3).

5. Raccord selon la revendication 4, caractérisé en ce que dans la cavité (3), la petite base forme une marche (17) ayant une hauteur comprise entre 1/10 et 3/10 du diamètre de la cavité (3).

6. Raccord selon l'une quelconque des revendications précédentes, caractérisé en ce que les ouvertures (11, 14) sont disposées dans deux manchons correspondants diamétralement opposés (9, 12) formés d'une seule pièce avec le corps (2), les axes des manchons (9, 12) étant perpendiculaires à l'axe de la cavité tubulaire (3).

7. Raccord selon la revendication 6, caractérisé en ce que la distance (S) entre les axes des manchons (9, 12) est comprise entre une et trois fois le diamètre de la cavité (3).

## Claims

1. A sampling and infusion connection for an extra-corporeal blood circulation line, comprising a body (2) having a tubular cavity (3), the two ends (4, 6) of which are adapted to be connected to two ducts (7, 8) belonging to the line, the body (2) also comprising an infusion opening (11) for introducing a liquid into the tubular cavity (3), and a sampling opening (14) for blood from the tubular cavity (3), the infusion opening (11) being situated downstream of the sampling opening (14) with respect to the blood flow, characterised in that the cavity (3) has a constriction (15) disposed between the openings (11, 14) and having a diameter which progressively decreases towards the infusion opening (11).

2. A connection according to claim 1, characterised in that the constriction (15) comprises at least a part of a Venturi tube.

3. A connection according to claim 1, characterised in that the constriction (15) has a frusto-conical surface (16), with its minor base substantially in line with the edge of the infusion opening (11).

4. A connection according to claim 3, characterised in that the frusto-conical surface (16) is inclined at an angle a between 10° and 20° relative to the axis of the cavity (3).

5. A connection according to claim 4, characterised in that in the cavity (3), the minor base forms a step (17) having a height between 1/10 and 3/10 of the diameter of the cavity (3).

6. A connection according to any of the preceding claims, characterised in that the apertures (11, 14) are disposed in two diametrically opposite corresponding sleeves (9, 12) formed in one piece with the body (2), the axes of the sleeves (9, 12) being perpendicular to the axis of the tubular cavity (3).

7. A connection according to claim 6, characterised in that the distance (S) between the axes of the sleeves (9, 12) is between one and three times the diameter of the cavity (3).

## Patentansprüche

1. Verbindungsstück für eine Leitung zur Blutzirkulation außerhalb des Körpers für eine Infusion und Probenentnahme, mit einem Korpus (2) mit einem rohrförmigen Hohlraum (3), dessen beide Enden (4, 6) dazu vorgesehen sind, mit zwei Schläuchen (7, 8) der Leitung verbunden zu werden, wobei der Korpus (2) auch eine Infusionsöffnung (11) zur Einführung einer Flüssigkeit in den rohrförmigen Hohlraum (3) und eine Öffnung (14) zur Probenentnahme von Blut von dem rohrförmigen Hohlraum (3) aus aufweist, wobei die Infusionsöffnung (11) stromabwärts der Probenentnahmeöffnung (14) bezüglich des Blutstroms angeordnet ist, **dadurch gekennzeichnet**, daß der Hohlraum (3) eine zwischen den Öffnungen (11, 14) angeordnete Einschnürung (15) aufweist, deren Durchmesser in Richtung auf die Infusionsöffnung (11) fortschreitend abnimmt.

2. Verbindungsstück nach Anspruch 1, **dadurch gekennzeichnet**, daß die Einschnürung (14) mindestens einen Teil eines Venturirohrs umfaßt.

3. Verbindungsstück nach Anspruch 1, **dadurch gekennzeichnet**, daß die Einschnürung (15) eine kegelstumpfförmige Fläche (16) aufweist, deren kleine Basis im wesentlichen mit dem Rand der Infusionsöffnung (11) fluchtet.

4. Verbindungsstück nach Anspruch 3, **dadurch gekennzeichnet**, daß die kegelstumpfförmige Fläche (16) unter einem Winkel α zwischen 10° und 20° hinsichtlich der Achse des Hohlraums (3) geneigt ist.

5. Verbindungsstück nach Anspruch 4, **dadurch gekennzeichnet**, daß in dem Hohlraum (3) die kleine Basis eine Stufe (17) mit einer Höhe zwischen 1/10 und 3/10 des Durchmessers des Hohlraums (3) bildet.

6. Verbindungsstück nach irgendeinem der vorhergehenden Ansprüche, **dadurch gekennzeichnet**, daß die Öffnungen (11, 14) in zwei einander diametral gegenüberliegenden entsprechenden Stutzen (9, 12) angeordnet sind, die einstückig mit dem Korpus (2) ausgebildet sind, wobei die Achsen der Stutzen (9, 12) rechtwinklig zur Achse des rohrförmigen Hohlraums (3) verlaufen.

7. Verbindungsstück nach Anspruch 6, **dadurch gekennzeichnet**, daß der Abstand (S) zwischen den Achsen der Stutzen (9, 12) zwischen dem Einfachen und dem Dreifachen des Durchmessers des Hohlraums (3) liegt.
